# EUROPEAN PATENT SPECIFICATION

(11) **EP 4 052 261 B1**
(45) Date of publication and mention of the grant of the patent: **04.03.2026**
(21) Application number: 20801355.7
(22) Date of filing: 23.10.2020
(51) Int. Cl.: G16C 20/10, G16C 20/50

(54) **DESIGNING A MOLECULE AND DETERMINING A ROUTE TO ITS SYNTHESIS**
ENTWURF EINES MOLEKÜLS UND BESTIMMUNG EINES WEGES ZU SEINER SYNTHESE
CONCEPTION D'UNE MOLÉCULE ET DÉTERMINATION D'UNE VOIE POUR SA SYNTHÈSE

(30) Priority: 28.10.2019 GB 201915623
(43) Date of publication of application: 07.09.2022
(73) Proprietor: BenevolentAI Technology Limited, London Greater London W1T 5HD (GB)
(72) Inventor: SEGLER, Martin, Hampshire PO5 2PR (GB); BROWN, Nathan, London Greater London SE19 2BY (GB)
(74) Representative: Gill Jennings & Every LLP
(86) International application number: PCT/GB2020/052702
(87) International publication number: WO 2021/084234

(56) References cited:
- WO-A2-2019/186196
- VAMATHEVAN JESSICA ET AL: "Applications of machine learning in drug discovery and development", NATURE REVIEWS DRUG DISCOVERY, NATURE PUBLISHING GROUP, GB, vol. 18, no. 6, 11 April 2019 (2019-04-11), pages 463 - 477, XP036796445, ISSN: 1474-1776, [retrieved on 20190411], DOI: 10.1038/S41573-019-0024-5
- PANTELEEV JANE ET AL: "Recent applications of machine learning in medicinal chemistry", BIORGANIC & MEDICINAL CHEMISTRY LETTERS, ELSEVIER, AMSTERDAM , NL, vol. 28, no. 17, 28 June 2018 (2018-06-28), pages 2807 - 2815, XP085447055, ISSN: 0960-894X, DOI: 10.1016/J.BMCL.2018.06.046
- MARCUS OLIVECRONA ET AL: "Molecular De Novo Design through Deep Reinforcement Learning", ARXIV.ORG, CORNELL UNIVERSITY LIBRARY, 201 OLIN LIBRARY CORNELL UNIVERSITY ITHACA, NY 14853, 25 April 2017 (2017-04-25), XP080765278, DOI: 10.1186/S13321-017-0235-X
- MARWIN H S SEGLER ET AL: "Learning to Plan Chemical Syntheses", ARXIV.ORG, CORNELL UNIVERSITY LIBRARY, 201 OLIN LIBRARY CORNELL UNIVERSITY ITHACA, NY 14853, 14 August 2017 (2017-08-14), XP081290729, DOI: 10.1038/NATURE25978

## Description

The present disclosure relates to systems and methods for designing a molecule or molecular structure and for determining viable routes to synthesis for the molecule. The presently disclosed techniques find particular application in the fields of biochemistry, drug discovery, agrochemistry, materials, fine chemicals, and fragrances.

### Background

In the fields of biochemistry, drug discovery, materials, agrochemistry, fine chemicals and fragrances, there is a need to design molecules with desired properties that make them suitable for use in particular applications and there is a need also to find suitable and practical ways to synthesise those molecules. A range of molecule design systems are currently available, as well as tools for determining viable routes to synthesis. Examples of such methods are described in AMATHEVAN JESSICA ET AL: "Applications of machine learning in drug discovery and development", NATURE REVIEWS DRUG DISCOVERY, NATURE PUBLISHING GROUP, GB, vol. 18, no. 6, (2019-04-11), pages 463-477. Such methods are also disclosed in OLIVECRONA, MARCUS ET AL: "Molecular De Novo Design through Deep Reinforcement Learning",ARXIV.ORG, 25 April 2017; in WO 2019/186196 A2; and in MARWIN H S SEGLER ET AL: "Learning to Plan Chemical Syntheses",ARXIV.ORG, 14 August 2017. However, these systems typically rely on a significant amount of input from the end-user who is generally a scientific expert in the field and is required to use his or her intuition or knowledge to direct, check or instruct various stages of the process. This breakdown of the process into user-dependent stages creates a burden on the end-user, introduces costs and delays into the process, and may bias the results in unforeseen ways.

In order to provide an improvement, a system is required that can reduce the reliance on input from the end-user and better support expert end-users in designing molecules and determining viable routes to synthesis.

The embodiments described below are not limited to implementations which solve any or all of the disadvantages of the known approaches described above.

### Summary

The invention is defined in the appended claims.

In a first aspect, the present invention provides a computer-implemented method of designing a molecule for use as a potential drug candidate and determining a route to synthesise the molecule, the method comprising: receiving one or more desired properties of the molecule; generating representations of one or more candidate molecules using a first machine learning technique that uses the one or more desired properties of the molecule as an input; and for each candidate molecule, computing one or more routes to synthesise the candidate molecule using a second machine learning technique that uses the representations of the candidate molecule and data relating to precursor molecules and reactions as inputs, wherein computing the one or more routes to synthesise each candidate molecule comprises exploring a reaction tree from the candidate molecule to precursor molecules using a tree search method, involving selecting and expanding nodes of the reaction tree by using a machine learning model trained to recognise valid chemical reactions; and outputting representations of candidate molecules and one or more associated routes to synthesis; the method further comprising: automatically providing feedback to the first machine learning technique indicating a suitability of one of the computed routes to synthesis in order to change the likelihood of future outputs of the first machine learning technique.

In a variation, the step of generating one or more candidate molecules may additionally or alternatively be performed using a chemoinformatics and/or artificial intelligence technique.

Optionally, the first machine learning technique comprises the use of generative adversarial networks variational autoencoders, recurrent neural networks, or genetic algorithms. Optionally, the method comprises ranking the candidate molecules based on at least one of the one or more desired properties. Optionally, exploring the reaction tree comprises using a Monte Carlo tree search method. Optionally, the method comprises generating the feedback by computing an evaluation of one of the candidate molecules and/or one of the computed routes to synthesis. Optionally, the method comprises failing to compute a route to synthesise one of the candidate molecules and feeding back an indication of the failure in order to reduce the likelihood of the candidate molecule being output in future. Optionally, the feedback is based on a user input. Optionally, the method comprises storing one or more of the computed routes as a macro action for use in a future synthesis route computation using the second machine learning technique. Optionally, the one or more desired properties of the molecule comprise one or more from the group of non-limiting examples consisting of solubility, toxicity, interaction with or binding to a target molecule or protein, blood brain barrier permeability, cell permeability, molecular similarity to extant molecules, physicochemical properties, ADMET characteristics, DMPK characteristics, docking scores, presence and characteristics of any toxicophores, whether the molecule is a controlled substance, presence of a pharmacophore, whether the molecule is novel, and whether the molecule is patented.

In a second aspect, the present invention provides a system for designing a molecule for use as a potential drug candidate and determining a route to synthesise the molecule, the system comprising: a molecular design module configured to: receive one or more desired properties of the molecule; and generate representations of one or more candidate molecules using a first machine learning technique that uses the one or more desired properties of the molecule as an input; and a synthesis route computation module configured to compute, for each candidate molecule, one or more routes to synthesise the candidate molecule using a second machine learning technique that uses the representations of the candidate molecule and data relating to precursor molecules and reactions as inputs, wherein computing the one or more routes to synthesise each candidate molecule comprises exploring a reaction tree from the candidate molecule to precursor molecules using a tree search method, involving selecting and expanding nodes of the reaction tree by using a machine learning model trained to recognise valid chemical reactions; wherein the system is configured to output a representation of candidate molecules and one or more associated routes to synthesis; the system further comprising: an evaluation module configured to automatically providing feedback to the first machine learning technique indicating a suitability of one of the computed routes to synthesis in order to change the likelihood of future outputs of the first machine learning technique.

Optionally, the first machine learning technique comprises the use of generative adversarial networks or variational autoencoders. Optionally, the system is configured to rank the candidate molecules based on one or more of the one or more desired properties.. Optionally, the system is configured to store one or more of the computed routes as a macro action for use in a future synthesis route computation using the second machine learning technique. Optionally, the one or more desired properties of the molecule comprise one or more from the group consisting of activity in a biochemical or phenotypic assay, solubility, toxicity, interaction with or binding to a target molecule or protein, blood brain barrier permeability, molecular similarity to extant molecules, physiochemical properties, ADMET characteristics, DMPK characteristics, docking scores, presence and characteristics of any toxicophores, whether the molecule is a controlled substance, presence of a pharmacophore, whether the molecule is novel, and whether the molecule is patented.

In a third aspect (not claimed), the present disclosure provides a computer-readable medium storing code that, when executed by a computer, causes the computer to perform the method of the first aspect.

The methods described herein may be performed by software in machine readable form on a tangible storage medium e.g. in the form of a computer program comprising computer program code means adapted to perform all the steps of any of the methods described herein when the program is run on a computer and where the computer program may be embodied on a computer readable medium. Examples of tangible (or non-transitory) storage media include disks, thumb drives, memory cards etc. and do not include propagated signals. The software can be suitable for execution on a parallel processor or a serial processor such that the method steps may be carried out in any suitable order, or simultaneously.

This application acknowledges that firmware and software can be valuable, separately tradable commodities. It is not intended to encompass software, which runs on or controls "dumb" or standard hardware, to carry out the desired functions. It is also not intended to encompass software which "describes" or defines the configuration of hardware, such as HDL (hardware description language) software, as is used for designing silicon chips, or for configuring universal programmable chips, to carry out desired functions.

The preferred features may be combined as appropriate, as would be apparent to a skilled person, and may be combined with any of the aspects of the invention.

### Brief Description of the Drawings

Embodiments of the invention will be described, by way of example, with reference to the following drawings, in which:
Figure 1 is a block diagram of a system for designing a molecule and for determining a route to synthesis for the molecule according to an embodiment of the invention;
Figure 2 is a flow chart of a method that may be carried out by the system;
Figure 3 is a block diagram of a molecular design module of the system showing optional features;
Figure 4 is a block diagram of a synthesis route computation module of the system showing optional features;
Figure 5 is a schematic diagram representing an example of a Monte Carlo Tree Search which may be used in accordance with the invention;
Figure 6 is a block diagram of the above showing additional optional features for providing feedback to the molecular design module and/or to the synthesis route computation module;
Figure 7 is a block diagram of a data store of the system showing optional features; and
Figure 8 is a block diagram of a computer suitable for implementing embodiments of the invention.

Common reference numerals are used throughout the figures to indicate similar features.

### Detailed Description

Embodiments of the present invention are described below by way of example only. These examples represent the best ways of putting the invention into practice that are currently known to the Applicant although they are not the only ways in which this could be achieved.

In the field of biochemistry, drug discovery, agrochemicals, materials, fine chemicals and fragrances, various techniques are available for designing molecules for specified purposes and for determining viable routes to synthesise them. Many of these techniques are automated, or partly automated, and use rule-based or machine learning approaches to solve aspects of the overall problem of molecule and synthesis route design. However, current approaches typically break the problem down into multiple stages, many of which require end-user input by a specialist scientist in order to direct, refine or otherwise guide the process to the next stage. This dependence on end-user input creates a burden on scientists' time and creates delays and increased costs of the end-to-end process.

The present inventors have appreciated that there is a need for a system that can design a molecule according to desired chemical or other properties and then supply routes to synthesis using available precursor compounds. As such, the inventors have developed an end-to-end system that designs both molecules and their synthesis routes automatically.

An end-to-end system is associated with a range of advantages. For example, plausible molecules that would have an excellent match with the desired properties but for which no viable route to synthesis can be determined can be ruled out from the start and do not need to be presented to the end-user as a possible result. Furthermore, a balance can be struck between the desirability of a molecule's properties and the ease with which it can be synthesised. As a result of this, a ranked set of molecules may be presented to an end-user that takes into account not only the extent to which the molecule meets the criteria of the desired properties, but also the relative ease or difficulty of its route or routes to synthesis. These advantages are not possible according to many typical approaches which separate out the design of a molecule from the subsequent determination of a viable route to synthesis.

In the present application, an end-to-end system is disclosed that includes a module for molecule design that uses machine learning techniques and another module for synthesis route computation which also uses machine learning techniques.

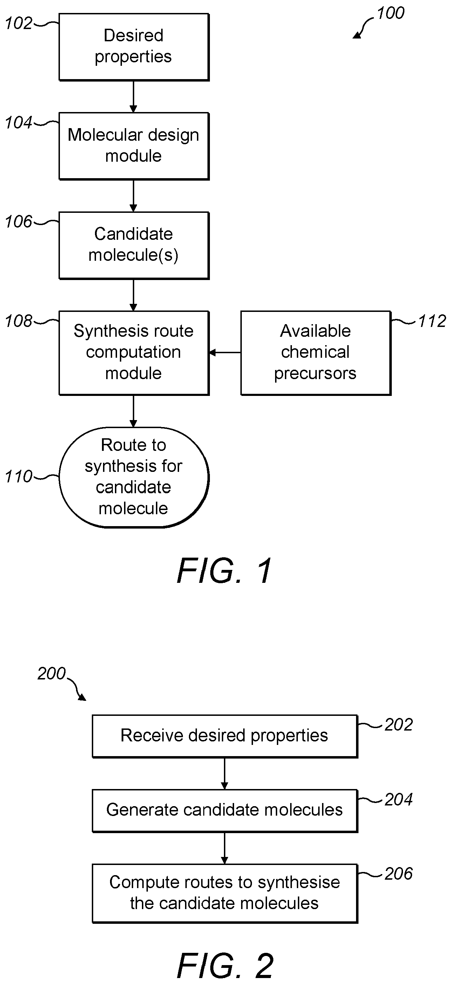

Figure 1 shows a system 100 for designing a molecule and determining routes for synthesising the molecule according to an embodiment of the invention. The system 100 is configured to receive as an input one or more desired properties 102 that the designed molecule is to possess or meet. For example, the one or more desired properties 102 may comprise chemical properties, physical, chemical or other constraints, or other requirements as further described below. These inputs 102 provide constraints which the system 100 is configured to apply in order to arrive at a suitable molecule or molecules before determining routes to synthesis. The one or more desired properties 102 may comprise a simple property requirement such as an acceptable solubility range. Alternatively, there may be multiple desired properties 102 which may, for example, be represented by a list or a data structure. If there are multiple desired properties 102, at least one of the desired properties 102 may be associated with a relative importance which may be included in a list or data structure representation.

The system comprises a molecular design module 104 which is configured to receive the one or more desired properties 102 and to generate, using a machine learning model, one or more candidate molecules 106 that match the one or more desired properties 102. The molecular design module 104 generates representations of the one or more candidate molecules 106 and, as shown in Figure 1, provides the representations as an input to a synthesis route computation module 108 of the system 100.

The synthesis route computation module 108 is configured to compute possible routes 110 to synthesise at least one candidate molecule 106, and in order to perform this computation it may have access to a dataset 112 of available chemical precursors that may be reacted in order to arrive at a candidate molecule 106. The final outcome is a representation of a molecule or molecules, alongside a route or routes that can be used to synthesise each molecule. As such, the system 100 is configured to output a representation of the one or more molecules and routes to synthesis. It will be appreciated that the system 100 may be configured such that if the synthesis route computation module 108 cannot find a route to synthesis for a candidate molecule, the system 100 excludes that candidate molecule from the output. Alternatively, the system 100 may be configured to output a molecule without a synthesis route if the synthesis route computation module 108 did not find a synthesis route for that molecule. In some examples (not claimed), the synthesis route computation module 108 is configured to compute synthesis routes only for one or more optimal candidate molecules, while according to the invention the synthesis route computation module 108 is configured to compute a synthesis route for each candidate molecule.

Accordingly, the present disclosure extends to a system for designing a molecule and determining a route to synthesise the molecule, the system comprising: a molecular design module 104 configured to: receive one or more desired properties of the molecule; and generate one or more candidate molecules using a first machine learning, chemoinformatics, computational and/or artificial intelligence technique that uses the one or more desired properties of the molecule as inputs; and a synthesis route computation module 108 configured to compute, for each candidate molecule, one or more routes to synthesise the candidate molecule using a second machine learning, chemoinformatics, computational and/or artificial intelligence technique that uses data relating to precursor molecules. In a variation, the molecule may be substituted for a molecular fragment such that the present disclosure also extends to a system for designing a molecular fragment and determining a route to synthesise the molecular fragment. Since the approach of the present disclosure traces the synthesis of each candidate molecule back to available chemical precursors via known reactions, it has the advantage of identifying one or more candidate molecules that are likely to be viable to synthesise in the lab. This breaks away from a mistaken assumption that it is sufficient to enumerate combinations of simpler molecular fragments to create a molecule that can be made in the lab. This assumption is not correct since even a combination of common molecular fragments does not guarantee synthesisability. As such, the approach of the present disclosure provides a technique for identifying molecules or molecular fragments with an improved rate of synthesisability in the lab.

The present disclosure also extends to a computer-implemented method 200 of designing a molecule and determining a route to synthesise the molecule, as shown in Figure 2. The method 200 comprises: receiving 202 one or more desired properties of the molecule; generating 204 one or more candidate molecules using a first machine learning technique that uses the one or more desired properties of the molecule as inputs; and for each candidate molecule, computing 206 one or more routes to synthesise the candidate molecule using a second machine learning technique that uses data relating to precursor molecules.

As indicated above, the molecular design module 104 is configured to receive as an input the one or more desired properties 102 that the molecule or molecules to be designed are required to possess or meet. The one or more desired properties 102 constrain the molecule design process and help to produce a molecule or molecules that closely match the desired criteria. A suitable example of a desired property 102 is that the molecule should be a potential drug candidate. Other non-limiting examples of desired properties 102 may comprise properties relating to solubility, toxicity, interaction with or binding to a target molecule or protein, or blood brain barrier permeability. Further non-limiting examples of desired properties 102 may relate to the following properties and characteristics.
- Efficacy, Affinity, Activity
- Molecular similarity to extant molecules
- Physiochemical properties such as molar weight (MW), logarithm of partition coefficient (CLogP), topological polar surface area (TPSA)
- Absorption, distribution, metabolism, excretion, toxicity (ADMET) characteristics
- Drug, metabolism and pharmacokinetics (DMPK) characteristics
- Docking scores in relation to other molecules
- Presence and characteristics of any toxicophores
- Whether the molecule is a controlled substance under relevant law
- Presence of a desired pharmacophore (which can be detected by pharmacophore matching techniques)
- Whether the molecule is novel
- Whether the molecule is patented
- Whether the molecule is disclosed in a published pending patent application

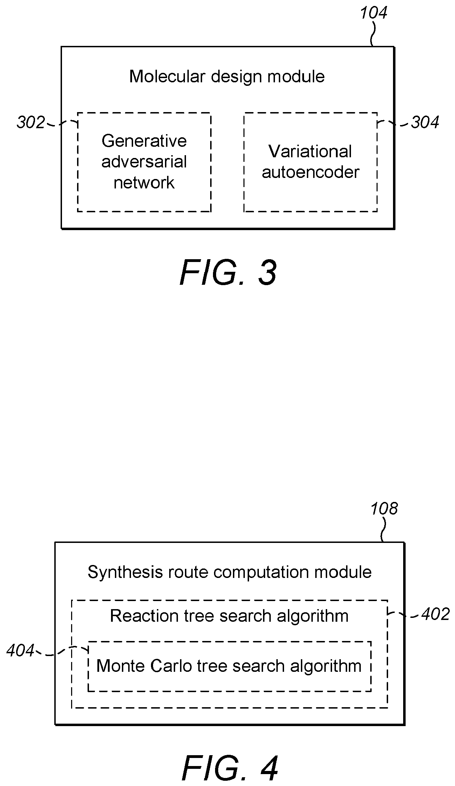

Referring to Figure 3, the molecular design module 104 is configured to receive representations of the one or more desired properties 102 and to design one or more suitable molecules that match the one or more desired properties 102 using a machine learning technique. The design process may comprise predicting and modelling biological activity, estimating prediction quality, or any other techniques that use learned properties to design potential output molecules. These may include the use of machine learning systems such as recurrent neural networks, transformers, generative adversarial networks, deep reinforcement learning agents, or variational autoencoders. As a result, in an embodiment of the invention the molecular design module 104 may comprise a generative adversarial network 302 and/or a variational autoencoder 304, as shown in Figure 3. The embodiment may additionally or alternatively comprise a neural network such as a recurrent neural network or an attention based neural network, a deep reinforcement learning agent, and/or a genetic algorithm. It will be appreciated that the machine learning model may be trained using, for example, unstructured data from relevant scientific literature or electronic notebook resources, and/or structured data from datasets such as chemical, biochemical or medical datasets.

The output of the molecule design module 104 comprises representations of one or more candidate molecules 106. The representations may, for example, comprise line notations such as SMILES chemical notation or international chemical identifier (InChl) text, or other suitable representations such as adjacency matrices or graphs.

The representations of the candidate molecules 106 generated by the molecular design module 104 are received as inputs by the synthesis route computation module 108 which is configured to compute routes to synthesis for each candidate molecule 106. This computation is achieved by the use of a machine learning technique that starts with a candidate molecule 106 and works backwards by performing a retrosynthetic analysis to determine how the molecule can be formed sequentially, in reverse order. As such, the synthesis route computation module 108 has access to a dataset 112 of available chemical precursor molecules from which potential routes to synthesis may be constructed and is trained to determine viable chemical reactions on the basis of training data comprising data such as known reaction tree data and chemical pathway data.

The machine learning technique used by the synthesis route computation module 108 involves conducting a search by expanding a tree of possible actions from the candidate molecule towards available chemical precursors. As such, the synthesis route computation module 108 is configured to compute one or more routes to synthesis by exploring a reaction tree from the candidate molecule to precursor molecules using a tree search method. The exploration involves selecting and expanding nodes of the reaction tree by using a machine learning model trained to recognise valid chemical reactions, and in this case the synthesis route computation module 108 comprises a reaction tree search algorithm 402 such as a Monte Carlo tree search algorithm 404, as shown in Figure 4. Other suitable examples of tree search methods that may be used by the synthesis route computation module 108 include A* search algorithms, Dijkstra's algorithm, and proof-number search and its variants.

In the example of the Monte Carlo tree search, the synthesis route computation module 108 comprises a Monte Carlo tree search retrosynthesis algorithm. In this approach, the root node of the tree search represents the final compound (i.e. the candidate molecule for which a synthesis route is to be found), and successive leaf nodes represent precursor compounds that can be reacted to produce the final compound. Monte Carlo tree search methods are advantageous for large action spaces (i.e. action spaces having high branching factors) as a result of their asymmetric growth. Such methods are also beneficially aheuristic and anytime. Selection and expansion of the leaf nodes involves the use of machine learning systems such as artificial neural networks that have been trained to recognise valid chemical reactions. Values are assigned to each node in the tree to represent the predicted value of further simulating the reaction pathway to which that node belongs, and decisions of which nodes to select may be implemented using various policies such as upper confidence-bounds for trees (UCT).

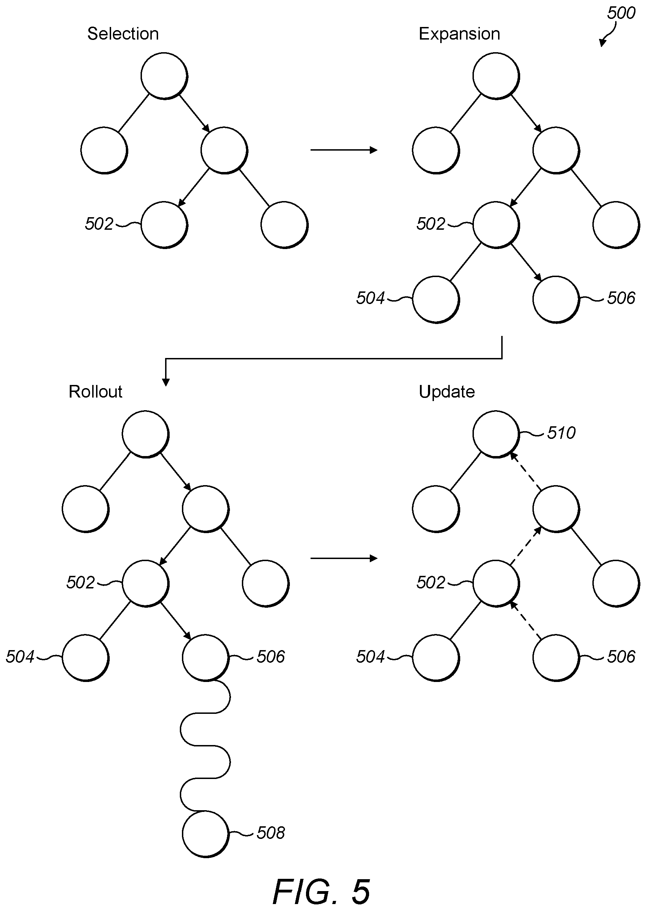

Figure 5 shows a schematic diagram of an example Monte Carlo tree search 500 which may be used by the synthesis route computation module 108. As shown, a promising node 502 for analysis is selected for expansion. The molecule represented by the node 502 is then processed by the machine learning system to generate precursor nodes 504 and 506 which represent valid chemical precursors. The most promising of these precursor nodes 506 is then selected for a rollout which generates a coarse prediction of the value of further expansion of that node 506. For example, the rollout may comprise a random sequence of valid reactions terminating in a node 508 which represents a precursor which is either known or for which no precursors are available. In this case, the random sequence of valid reactions is used to generate a prediction of the value of further expansion of the node 506, and this value is backpropagated from the node 506 back to the root node 510, updating relevant scores of each node along the route. A number of promising nodes may be simulated in this way, and their predicted values backpropagated to the root node 510 to update the tree. In this way, once a number of simulations have been performed on promising precursor nodes, the computation may terminate and return the most promising route to synthesis for the candidate molecule from the available precursors.

The synthesis route computation module 108 may be configured to perform multiple searches with a view to returning multiple routes to synthesis for each candidate molecule 106, and is configured to provide as outputs candidate molecules together with their respective route or routes to synthesis. If there are multiple candidate molecules 106 each having at least one route to synthesis, the system 100 may be configured to rank the candidate molecules 106 based on at least one of the one or more desired properties 102 or based on a metric derived from at least one of the one or more desired properties 102. For example, candidate molecules 106 may be ranked by toxicity, complexity to synthesise, and closeness to at least one of the one or more desired properties 102.

In any case, the system 100 is configured to output representations of candidate molecules 106 and their routes to synthesis. If there is a candidate molecule 106 for which no route to synthesis can be found, this candidate molecule may be excluded from the set of output results.

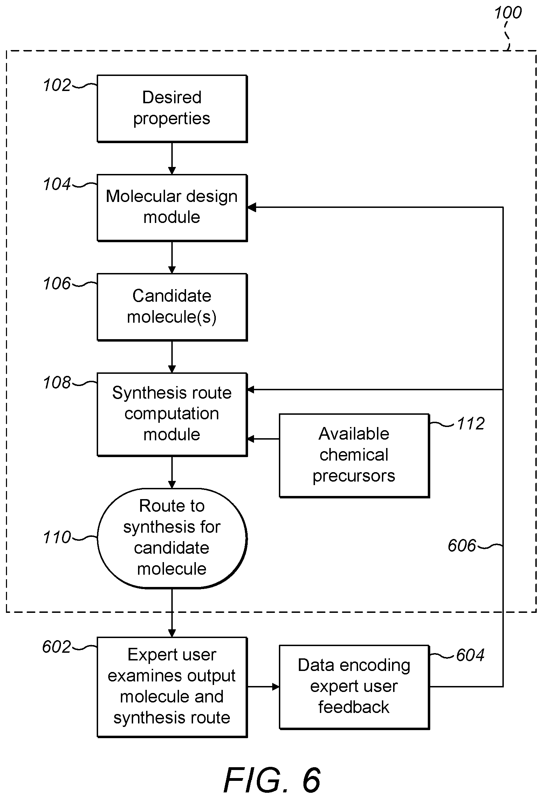

In some cases (not claimed), an end-user may review the outputs and provide feedback to the system 100 as to the suitability of the molecules and/or the routes to synthesis based on his or her expert knowledge and experience. In this case, the system 100 is configured as described above, but in addition an expert end-user may examine a representation of an output molecule and/or synthesis route (at block 602) and provide an associated user input, as shown in Figure 6. The user input may provide information relating to whether the end-user considers the molecule to be a reasonable candidate with respect to the one or more desired properties and/or whether the end-user considers the route to synthesis to be physically possible or practicable.

The user input containing the feedback may be encoded into a data format 604 suitable for feeding back 606 to one or both of the molecular design module 104 and the synthesis route computation module 108. In this way, the respective machine learning models of the molecular design module 104 and the synthesis route computation module 108 may learn to prioritise more suitable candidate molecules that are more likely to meet the desired chemical properties or are more practical to synthesise or both. Such feedback may also reduce the risk of a molecule being designed that cannot successfully be synthesised.

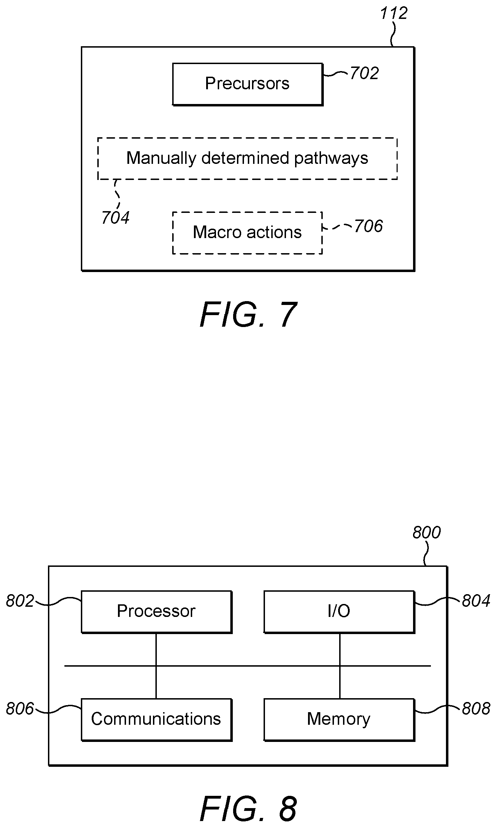

As shown in Figure 7, in some embodiments the dataset 112 of available chemical precursors stores not only precursors 702, but also manually determined pathways 704 which may, for example, be determined by scientific experts and may be used in synthesis route computations (as well as in the training data for the synthesis route computation module 108). Synthesis routes that are generated by the synthesis route computation module 108 may also be stored in the dataset 112 as macro actions 706 for re-use in future synthesis route computations. The re-use of the macro actions 706 advantageously grows the training set with each iteration of the synthesis route computation module 108.

In other embodiments (not all of which are claimed), the feedback may be generated automatically by the system 100. In this case, the system 100 may comprise an evaluation module configured to compute an evaluation of one of the candidate molecules, one of the routes to synthesis, or both, and to provide the evaluation as feedback to the first machine learning technique and/or the second machine learning technique in order to change the likelihood of future outputs of the first machine learning technique or the second machine learning technique or both. In cases where the synthesis route computation module 108 fails to produce a synthesis route for a candidate molecule, for example because such a synthesis route does not exist or because the synthesis route computation module is unable to generate such a route, an indication of this failure may be fed back to the first machine learning technique in order to reduce the likelihood of the molecular design module 104 outputting that molecule in future. According to the invention, feedback indicating a suitability of one of the computed routes to synthesis is provided to the first machine learning technique automatically in order to change the likelihood of future outputs of the first machine learning technique.

A computer apparatus 800 suitable for implementing methods according to the present invention is shown in Figure 8. The apparatus 800 comprises a processor 802, an input-output device 804, a communications portal 806 and computer memory 808. The memory 808 may store code that, when executed by the processor 802, causes the apparatus 800 to perform the method 200 shown in Figure 2.

In the embodiment described above the server may comprise a single server or network of servers. In some examples the functionality of the server may be provided by a network of servers distributed across a geographical area, such as a worldwide distributed network of servers, and a user may be connected to an appropriate one of the network of servers based upon a user location.

The above description discusses embodiments of the invention with reference to a single user for clarity. It will be understood that in practice the system may be shared by a plurality of users, and possibly by a very large number of users simultaneously.

The embodiments described above are fully automatic. In some examples a user or operator of the system may manually instruct some steps of the method to be carried out.

In the described embodiments of the invention the system may be implemented as any form of a computing and/or electronic device. Such a device may comprise one or more processors which may be microprocessors, controllers or any other suitable type of processors for processing computer executable instructions to control the operation of the device in order to gather and record routing information. In some examples, for example where a system on a chip architecture is used, the processors may include one or more fixed function blocks (also referred to as accelerators) which implement a part of the method in hardware (rather than software or firmware). Platform software comprising an operating system or any other suitable platform software may be provided at the computing-based device to enable application software to be executed on the device.

Various functions described herein can be implemented in hardware, software, or any combination thereof. If implemented in software, the functions can be stored on or transmitted over as one or more instructions or code on a computer-readable medium. Computer-readable media may include, for example, computer-readable storage media. Computer-readable storage media may include volatile or non-volatile, removable or non-removable media implemented in any method or technology for storage of information such as computer readable instructions, data structures, program modules or other data. A computer-readable storage media can be any available storage media that may be accessed by a computer. By way of example, and not limitation, such computer-readable storage media may comprise RAM, ROM, EEPROM, flash memory or other memory devices, CD-ROM or other optical disc storage, magnetic disc storage or other magnetic storage devices, or any other medium that can be used to carry or store desired program code in the form of instructions or data structures and that can be accessed by a computer. Disc and disk, as used herein, include compact disc (CD), laser disc, optical disc, digital versatile disc (DVD), floppy disk, and bluray disc (BD). Further, a propagated signal is not included within the scope of computer-readable storage media. Computer-readable media also includes communication media including any medium that facilitates transfer of a computer program from one place to another. A connection, for instance, can be a communication medium. For example, if the software is transmitted from a website, server, or other remote source using a coaxial cable, fiber optic cable, twisted pair, DSL, or wireless technologies such as infrared, radio, and microwave are included in the definition of communication medium. Combinations of the above should also be included within the scope of computer-readable media.

Alternatively, or in addition, the functionality described herein can be performed, at least in part, by one or more hardware logic components. For example, and without limitation, hardware logic components that can be used may include Field-Programmable Gate Arrays (FPGAs), Program-Specific Integrated Circuits (ASICs), Program-Specific Standard Products (ASSPs), System-On-a-Chip systems (SOCs), Complex Programmable Logic Devices (CPLDs), etc.

Although illustrated as a single system, it is to be understood that the computing device may be a distributed system. Thus, for instance, several devices may be in communication by way of a network connection and may collectively perform tasks described as being performed by the computing device.

Although illustrated as a local device it will be appreciated that the computing device may be located remotely and accessed via a network or other communication link (for example using a communication interface).

The term 'computer' is used herein to refer to any device with processing capability such that it can execute instructions. Those skilled in the art will realise that such processing capabilities are incorporated into many different devices and therefore the term 'computer' includes PCs, servers, mobile telephones, personal digital assistants and many other devices.

Those skilled in the art will realise that storage devices utilised to store program instructions can be distributed across a network. For example, a remote computer may store an example of the process described as software. A local or terminal computer may access the remote computer and download a part or all of the software to run the program. Alternatively, the local computer may download pieces of the software as needed, or execute some software instructions at the local terminal and some at the remote computer (or computer network). Those skilled in the art will also realise that by utilising conventional techniques known to those skilled in the art that all, or a portion of the software instructions may be carried out by a dedicated circuit, such as a DSP, programmable logic array, or the like.

It will be understood that the benefits and advantages described above may relate to one embodiment or may relate to several embodiments. The embodiments are not limited to those that solve any or all of the stated problems or those that have any or all of the stated benefits and advantages.

Any reference to "an" item refers to one or more of those items. The term 'comprising' is used herein to mean including the method steps or elements identified, but that such steps or elements do not comprise an exclusive list and a method or apparatus may contain additional steps or elements.

As used herein, the terms "component" and "system" are intended to encompass computer-readable data storage that is configured with computer-executable instructions that cause certain functionality to be performed when executed by a processor. The computer-executable instructions may include a routine, a function, or the like. It is also to be understood that a component or system may be localized on a single device or distributed across several devices.

Further, as used herein, the term "exemplary" is intended to mean "serving as an illustration or example of something".

Further, to the extent that the term "includes" is used in either the detailed description or the claims, such term is intended to be inclusive in a manner similar to the term "comprising" as "comprising" is interpreted when employed as a transitional word in a claim.

Moreover, the acts described herein may comprise computer-executable instructions that can be implemented by one or more processors and/or stored on a computer-readable medium or media. The computer-executable instructions can include routines, sub-routines, programs, threads of execution, and/or the like. Still further, results of acts of the methods can be stored in a computer-readable medium, displayed on a display device, and/or the like.

It will be understood that the above description of a preferred embodiment is given by way of example only and that various modifications may be made by those skilled in the art. The invention, however, is defined in the appended claims.

## Claims

1. A computer-implemented method of designing a molecule for use as a potential drug candidate and determining a route to synthesise the molecule, the method comprising:
receiving one or more desired properties of the molecule;
generating representations of one or more candidate molecules using a first machine learning technique that uses the one or more desired properties of the molecule as an input; and
for each candidate molecule, computing one or more routes to synthesise the candidate molecule using a second machine learning technique that uses the representations of the candidate molecule and data relating to precursor molecules and reactions as inputs, wherein computing the one or more routes to synthesise each candidate molecule comprises exploring a reaction tree from the candidate molecule to precursor molecules using a tree search method, involving selecting and expanding nodes of the reaction tree by using a machine learning model trained to recognise valid chemical reactions; and
outputting representations of candidate molecules and one or more associated routes to synthesis; the method further comprising:
automatically providing feedback to the first machine learning technique indicating a suitability of one of the computed routes to synthesis in order to change the likelihood of future outputs of the first machine learning technique.

2. The computer-implemented method of claim 1, wherein the first machine learning technique comprises the use of generative adversarial networks, variational autoencoders, recurrent neural networks or genetic algorithms.

3. The computer-implemented method of claim 1 or 2, comprising ranking the candidate molecules based on at least one of the one or more desired properties.

4. The computer-implemented method of any preceding claim, wherein exploring the reaction tree comprises using a Monte Carlo tree search method in which a root node of the tree search represents the candidate molecule and successive leaf nodes represent precursor compounds that can be reacted to produce the candidate molecule.

5. The computer-implemented method of claim 4, where the method comprises:
selecting a promising precursor node and generating a random sequence of valid reactions terminating in a node which represents a precursor that is known or for which no precursors are available, thereby generating a coarse prediction of the value of further expanding the node;
backpropagating the prediction to the root node to update the reaction tree.

6. The computer-implemented method of any preceding claim, comprising failing to compute a route to synthesise one of the candidate molecules and feeding back an indication of the failure in order to reduce the likelihood of the candidate molecule being output in future.

7. The computer-implemented method of any preceding claim, comprising storing one or more of the computed routes as a macro action for use in a future synthesis route computation using the second machine learning technique.

8. The computer-implemented method of any preceding claim, wherein the one or more desired properties of the molecule comprise one or more from the group consisting of solubility, toxicity, efficacy, activity in a phenotypic or biochemical assay, interaction with or binding to a target molecule or protein, blood brain barrier permeability, molecular similarity to extant molecules, physicochemical properties, ADMET characteristics, DMPK characteristics, docking scores, presence and characteristics of any toxicophores, whether the molecule is a controlled substance, presence of a pharmacophore, whether the molecule is novel, and whether the molecule is patented.

9. A system (100) for designing a molecule for use as a potential drug candidate and determining a route to synthesise the molecule, the system comprising:
a molecular design module (104) configured to:
receive one or more desired properties (102) of the molecule; and
generate representations of one or more candidate molecules using a first machine learning technique that uses the one or more desired properties of the molecule as an input; and
a synthesis route computation module (108) configured to compute, for each candidate molecule, one or more routes to synthesise the candidate molecule using a second machine learning technique that uses the representations of the candidate molecule and data relating to precursor molecules and reactions as inputs, wherein computing the one or more routes to synthesise each candidate molecule comprises exploring a reaction tree from the candidate molecule to precursor molecules using a tree search method, involving selecting and expanding nodes of the reaction tree by using a machine learning model trained to recognise valid chemical reactions;
wherein the system is configured to output a representation of candidate molecules and one or more associated routes to synthesis; the system further comprising:
an evaluation module configured to automatically providing feedback to the first machine learning technique indicating a suitability of one of the computed routes to synthesis in order to change the likelihood of future outputs of the first machine learning technique .

10. The system of claim 9, wherein the first machine learning technique comprises the use of generative adversarial networks or variational autoencoders.

11. The system of claim 9 or 10, configured to rank the candidate molecules based on one or more of the one or more desired properties.

12. The system of any of claims 9 to 11, configured to store one or more of the computed routes as a macro action for use in a future synthesis route computation using the second machine learning technique.

13. The system of any of claims 9 to 12, wherein the one or more desired properties of the molecule comprise one or more from the group consisting of solubility, toxicity, interaction with or binding to a target molecule or protein, blood brain barrier permeability, molecular similarity to extant molecules, physicochemical properties, ADMET characteristics, DMPK characteristics, docking scores, presence and characteristics of any toxicophores, whether the molecule is a controlled substance, presence of a pharmacophore, whether the molecule is novel, and whether the molecule is patented.

## Patentansprüche

1. Computerimplementiertes Verfahren zum Entwerfen eines Moleküls, um es als potenziellen Kandidaten für ein Medikament zu verwenden und um einen Weg zur Synthese des Moleküls zu bestimmen, das Verfahren umfassend:
Erhalten einer oder mehrerer gewünschter Eigenschaften des Moleküls;
Erzeugen von Darstellungen einer oder mehrerer Kandidatenmoleküle unter Verwendung einer ersten maschinellen Lerntechnik, die die eine oder mehreren gewünschten Eigenschaften des Moleküls als Eingabe verwendet; und
für jedes Kandidatenmolekül das Berechnen eines oder mehrerer Wege zur Synthese des Kandidatenmoleküls unter Verwendung einer zweiten maschinellen Lerntechnik, die die Darstellungen des Kandidatenmoleküls und Daten in Bezug auf Vorläufermoleküle und Reaktionen als Eingaben verwendet, wobei das Berechnen des einen oder der mehreren Wege zur Synthese jedes Kandidatenmoleküls das Erforschen eines Reaktionsbaums von dem Kandidatenmolekül zu Vorläufermolekülen unter Verwendung eines Verfahrens zur Baumsuche umfasst, um Knoten des Reaktionsbaums unter Verwendung eines maschinellen Lernmodells auszuwählen und zu erweitern, das darauf trainiert ist, gültige chemische Reaktionen zu erkennen; und
Ausgeben von Darstellungen von Kandidatenmolekülen und einem oder mehreren zugehörigen Synthesewegen; das Verfahren weiter umfassend:
automatisches Bereitstellen von Feedback an die erste maschinelle Lerntechnik, das die Eignung einer der berechneten Wege für die Synthese angibt, um die Wahrscheinlichkeit zukünftiger Ergebnisse der ersten maschinellen Lerntechnik zu ändern.

2. Computerimplementiertes Verfahren nach Anspruch 1, wobei die erste maschinelle Lerntechnik die Verwendung von generativen gegnerischen Netzwerken, variationalen Autoencodern, rekurrenten neuronalen Netzwerken oder genetischen Algorithmen umfasst.

3. Computerimplementierte Verfahren nach Anspruch 1 oder 2, umfassend das Einstufen der Kandidatenmoleküle basierend auf mindestens einer der einen oder mehreren gewünschten Eigenschaften.

4. Computerimplementiertes Verfahren nach einem der vorstehenden Ansprüche, wobei das Erforschen des Reaktionsbaums die Verwendung eines Monte-Carlo-Baum-Verfahrens umfasst, bei dem ein Wurzelknoten der Baumsuche das Kandidatenmolekül darstellt und aufeinanderfolgende Blattknoten Vorläuferverbindungen darstellen, die zur Erzeugung des Kandidatenmoleküls reagieren können.

5. Computerimplementiertes Verfahren nach Anspruch 4, wobei das Verfahren Folgendes umfasst:
Auswählen eines vielversprechenden Vorläuferknotens und Erzeugen einer zufälligen Sequenz gültiger Reaktionen, die in einem Knoten enden, der einen bekannten Vorläufer darstellt oder für den keine Vorläufer verfügbar sind, wodurch eine grobe Vorhersage des Werts einer weiteren Erweiterung des Knotens erzeugt wird;
Rückübertragen der Vorhersage an den Wurzelknoten, um den Reaktionsbaum zu aktualisieren.

6. Computerimplementiertes Verfahren nach einem der vorstehenden Ansprüche, umfassend das Nichtberechnen eines Wegs zur Synthese eines der Kandidatenmoleküle und das Zurückmelden einer Anzeige des Fehlschlags, um die Wahrscheinlichkeit zu verringern, dass das Kandidatenmolekül in Zukunft ausgegeben wird.

7. Computerimplementiertes Verfahren nach einem der vorstehenden Ansprüche, umfassend das Speichern einer oder mehrerer der berechneten Wege als Makroaktion, um sie bei einer zukünftigen Synthesewegeberechnung zu verwenden, unter Verwendung der zweiten maschinellen Lerntechnik.

8. Computerimplementiertes Verfahren nach einem der vorstehenden Ansprüche, wobei die eine oder mehrere gewünschten Eigenschaften des Moleküls eine oder mehrere aus der Gruppe bestehend aus Löslichkeit, Toxizität, Wirksamkeit, Aktivität in einem phänotypischen oder biochemischen Assay, Wechselwirkung mit oder Bindung an ein Zielmolekül oder -protein, Durchlässigkeit der Blut-Hirn-Schranke, molekulare Ähnlichkeit mit vorhandenen Molekülen, physikalisch-chemische Eigenschaften, ADMET-Eigenschaften, DMPK-Eigenschaften, Docking-Scores, Vorhandensein und Eigenschaften von Toxikophoren, ob das Molekül eine kontrollierte Substanz ist, Vorhandensein eines Pharmakophors, ob das Molekül neuartig ist und ob das Molekül patentiert ist, umfasst.

9. System (100) zum Entwerfen eines Moleküls, um es als potenziellen Kandidaten für ein Medikament zu verwenden und um einen Weg zur Synthese des Moleküls zu bestimmen, das System umfassend:
ein Moleküldesignmodul (104), das konfiguriert ist, um:
eine oder mehrere gewünschte Eigenschaften (102) des Moleküls zu empfangen; und
eine oder mehrere Darstellungen von Kandidatenmolekülen unter Verwendung einer ersten maschinellen Lerntechnik zu erzeugen, die die eine oder mehrere gewünschten Eigenschaften des Moleküls als Eingabe verwendet; und
ein Syntheseweg-Berechnungsmodul (108), das konfiguriert ist, um für jedes Kandidatenmolekül einen oder mehrere Wege zur Synthese des Kandidatenmoleküls unter Verwendung einer zweiten maschinellen Lerntechnik zu berechnen, die die Darstellungen des Kandidatenmoleküls und Daten in Bezug auf Vorläufermoleküle und Reaktionen als Eingaben verwendet, wobei das Berechnen des einen oder der mehreren Wege zur Synthese jedes Kandidatenmoleküls das Erforschen eines Reaktionsbaums von dem Kandidatenmolekül zu Vorläufermolekülen unter Verwendung eines Verfahrens zur Baumsuche umfasst, um Knoten des Reaktionsbaums unter Verwendung eines maschinellen Lernmodells auszuwählen und zu erweitern, das darauf trainiert ist, gültige chemische Reaktionen zu erkennen;
wobei das System so konfiguriert ist, dass es eine Darstellung von Kandidatenmolekülen und einen oder mehrere zugehörige Synthesewege ausgibt; das System weiter umfassend:
ein Bewertungsmodul, das so konfiguriert ist, dass es automatisch Feedback an die erste maschinelle Lerntechnik liefert, die die Eignung eines der berechneten Synthesewege angibt, um die Wahrscheinlichkeit zukünftiger Ausgaben der ersten maschinellen Lerntechnik zu ändern.

10. System nach Anspruch 9, wobei die erste maschinelle Lerntechnik die Verwendung von generativen gegnerischen Netzwerken oder variationalen Autoencodern umfasst.

11. System nach Anspruch 9 oder 10, das konfiguriert ist, um die Kandidatenmoleküle basierend auf einer oder mehrerer der gewünschten Eigenschaften zu bewerten.

12. System nach einem der Ansprüche 9 bis 11, das konfiguriert ist, um einen oder mehrere der berechneten Wege als Makroaktion zu speichern, die bei einer zukünftigen Synthese-Wegberechnung unter Verwendung der zweiten maschinellen Lerntechnik verwendet werden kann.

13. System nach einem der Ansprüche 9 bis 12, wobei die eine oder mehrere gewünschten Eigenschaften des Moleküls eine oder mehrere aus der Gruppe bestehend aus Löslichkeit, Toxizität, Wechselwirkung mit oder Bindung an ein Zielmolekül oder - protein, Durchlässigkeit der Blut-Hirn-Schranke, molekulare Ähnlichkeit mit vorhandenen Molekülen, physikalisch-chemische Eigenschaften, ADMET-Eigenschaften, DMPK-Eigenschaften, Docking-Scores, Vorhandensein und Eigenschaften von Toxikophoren, ob das Molekül eine kontrollierte Substanz ist, Vorhandensein eines Pharmakophors, ob das Molekül neuartig ist und ob das Molekül patentiert ist, umfasst.

## Revendications

1. Procédé mis en œuvre par ordinateur de conception d'une molécule pour une utilisation en tant que médicament candidat potentiel et de détermination d'une voie de synthèse de la molécule, le procédé comprenant :
la réception d'une ou de plusieurs propriétés souhaitées de la molécule ;
la génération de représentations d'une ou de plusieurs molécules candidates à l'aide d'une première technique d'apprentissage automatique qui utilise les une ou plusieurs propriétés souhaitées de la molécule en entrée ; et
pour chaque molécule candidate, le calcul d'une ou de plusieurs voies de synthèse de la molécule candidate à l'aide d'une seconde technique d'apprentissage automatique qui utilise les représentations de la molécule candidate et des données relatives à des molécules et des réactions de précurseurs en entrées,
dans lequel le calcul des une ou plusieurs voies de synthèse de chaque molécule candidate comprend l'exploration d'une arborescence de réaction de la molécule candidate à des molécules de précurseur à l'aide d'un procédé de recherche arborescente, impliquant la sélection et l'expansion de nœuds de l'arborescence de réaction en utilisant un modèle d'apprentissage automatique entraîné à reconnaître des réactions chimiques valides ; et
la sortie de représentations de molécules candidates et d'une ou de plusieurs voies de synthèse associées ; le procédé comprenant en outre :
la fourniture de manière automatique d'un renvoi d'informations à la première technique d'apprentissage automatique indiquant une adéquation de l'une des voies de synthèse calculées afin de modifier la probabilité de sorties futures de la première technique d'apprentissage automatique.

2. Procédé mis en œuvre par ordinateur selon la revendication 1, dans lequel la première technique d'apprentissage automatique comprend l'utilisation de réseaux antagonistes génératifs, d'auto-encodeurs variationnels, de réseaux de neurones récurrents ou d'algorithmes génétiques.

3. Procédé mis en œuvre par ordinateur selon la revendication 1 ou la revendication 2, comprenant le classement des molécules candidates sur la base d'au moins l'une parmi les une ou plusieurs propriétés souhaitées.

4. Procédé mis en œuvre par ordinateur selon une quelconque revendication précédente, dans lequel l'exploration de l'arborescence de réaction comprend l'utilisation d'un procédé de recherche arborescente de Monte Carlo dans lequel un nœud racine de la recherche arborescente représente la molécule candidate et des nœuds feuilles successifs représentent des composés précurseurs qui peuvent être mis en réaction pour produire la molécule candidate.

5. Procédé mis en œuvre par ordinateur selon la revendication 4, dans lequel le procédé comprend :
la sélection d'un nœud précurseur prometteur et la génération d'une séquence aléatoire de réactions valides se terminant dans un nœud qui représente un précurseur qui est connu ou pour lequel aucun précurseur n'est disponible, ce qui génère une prédiction grossière de la valeur d'une expansion supplémentaire du nœud ;
la rétropropagation de la prédiction jusqu'au nœud racine pour mettre à jour l'arborescence de réaction.

6. Procédé mis en œuvre par ordinateur selon une quelconque revendication précédente, comprenant le fait d'échouer à calculer une voie de synthèse de l'une des molécules candidates et le renvoi d'une indication de l'échec afin de réduire la probabilité que la molécule candidate soit délivrée en sortie dans le futur.

7. Procédé mis en œuvre par ordinateur selon une quelconque revendication précédente, comprenant le stockage de l'une ou de plusieurs des voies calculées sous la forme d'une macro-action pour une utilisation dans un futur calcul de voie de synthèse à l'aide de la seconde technique d'apprentissage automatique.

8. Procédé mis en œuvre par ordinateur selon une quelconque revendication précédente, dans lequel les une ou plusieurs propriétés souhaitées de la molécule comprennent l'un ou plusieurs parmi le groupe consistant en la solubilité, la toxicité, l'efficacité, l'activité dans un dosage phénotypique ou biochimique, l'interaction avec ou la liaison à une molécule ou protéine cible, la perméabilité de la barrière hématoencéphalique, la similarité moléculaire avec des molécules existantes, les propriétés physicochimiques, les caractéristiques ADMET, les caractéristiques DMPK, les scores d'accrochage, la présence et les caractéristiques de tout toxicophore, le fait de savoir si la molécule est une substance réglementée, la présence d'un pharmacophore, le fait de savoir si la molécule est nouvelle, et le fait de savoir si la molécule est brevetée.

9. Système (100) pour la conception d'une molécule pour une utilisation en tant que médicament candidat potentiel et de détermination d'une voie de synthèse de la molécule, le système comprenant :
un module (104) de conception moléculaire configuré pour :
recevoir une ou plusieurs propriétés (102) souhaitées de la molécule ; et
générer des représentations d'une ou de plusieurs molécules candidates à l'aide d'une première technique d'apprentissage automatique qui utilise les une ou plusieurs propriétés souhaitées de la molécule en entrée ; et
un module (108) de calcul de voie de synthèse configuré pour calculer, pour chaque molécule candidate, une ou plusieurs voies de synthèse de la molécule candidate à l'aide d'une seconde technique d'apprentissage automatique qui utilise les représentations de la molécule candidate et des données relatives à des molécules et des réactions de précurseurs en entrées, dans lequel le calcul des une ou plusieurs voies de synthèse de chaque molécule candidate comprend l'exploration d'une arborescence de réaction de la molécule candidate à des molécules de précurseur à l'aide d'un procédé de recherche arborescente, impliquant la sélection et l'expansion de nœuds de l'arborescence de réaction en utilisant un modèle d'apprentissage automatique entraîné à reconnaître des réactions chimiques valides ;
dans lequel le système est configuré pour délivrer en sortie une représentation de molécules candidates et une ou plusieurs voies de synthèse associées ; le système comprenant en outre :
un module d'évaluation configuré pour fournir de manière automatique un renvoi d'informations à la première technique d'apprentissage automatique indiquant une adéquation de l'une des voies de synthèse calculées afin de modifier la probabilité de sorties futures de la première technique d'apprentissage automatique.

10. Système selon la revendication 9, dans lequel la première technique d'apprentissage automatique comprend l'utilisation de réseaux antagonistes génératifs ou d'auto-encodeurs variationnels.

11. Système selon la revendication 9 ou la revendication 10, configuré pour classer les molécules candidates sur la base de l'une ou de plusieurs propriétés parmi les une ou plusieurs propriétés souhaitées.

12. Système selon l'une quelconque des revendications 9 à 11, configuré pour stocker l'une ou de plusieurs des voies calculées sous la forme d'une macro-action pour une utilisation dans un futur calcul de voie de synthèse à l'aide de la seconde technique d'apprentissage automatique.

13. Système selon l'une quelconque des revendications 9 à 12, dans lequel les une ou plusieurs propriétés souhaitées de la molécule comprennent l'un ou plusieurs parmi le groupe consistant en la solubilité, la toxicité, l'interaction avec ou la liaison à une molécule ou protéine cible, la perméabilité de la barrière hématoencéphalique, la similarité moléculaire avec des molécules existantes, les propriétés physicochimiques, les caractéristiques ADMET, les caractéristiques DMPK, les scores d'accrochage, la présence et les caractéristiques de tout toxicophore, le fait de savoir si la molécule est une substance réglementée, la présence d'un pharmacophore, le fait de savoir si la molécule est nouvelle, et le fait de savoir si la molécule est brevetée.
